# EUROPEAN PATENT APPLICATION

(11) **EP 4 851 274 A1**
(43) Date of publication of application: **09.12.2026**
(21) Application number: 25748698.5
(22) Date of filing: 30.01.2025
(51) Int. Cl.: C12M 3/00, C12M 1/34, C12N 5/071

(54) **CELL CULTURE DEVICE AND CELL CULTURE METHOD USING SAME**

(30) Priority: 01.02.2024 JP 2024013960
(71) Applicant: Institute of Science Tokyo, Tokyo 152-8550 (JP); Nepa Gene Co., Ltd., Ichikawa-shi, Chiba 272-0114 (JP)
(72) Inventor: MAKANAE, Aki, Ichikawa-shi, Chiba 272-0114 (JP); SUZUKI, Kosho, Ichikawa-shi, Chiba 272-0114 (JP); KURIHARA, Kinya, Ichikawa-shi, Chiba 272-0114 (JP); NAKAMOTO, Kazuki, Ichikawa-shi, Chiba 272-0114 (JP); YASHIRO, Mitsuru, Ichikawa-shi, Chiba 272-0114 (JP); IWAMOTO, Tsutomu, Tokyo 152-8550 (JP); KAWASHIMA, Nobuyuki, Tokyo 152-8550 (JP); SUGIMOTO, Asuna, Tokyo 152-8550 (JP); OHNISHI, Kayoko, Tokyo 152-8550 (JP)
(74) Representative: Plougmann Vingtoft a/s
(86) International application number: PCT/JP2025/002887
(87) International publication number: WO 2025/164695

(57) **Abstract**

An apparatus for culturing cells capable of applying one or more physical and/or chemical stimuli to cells during culture, and a method of culturing cells using the same are disclosed. The apparatus for culturing cells includes a region for culturing cells, a first pump connected to the region for culturing cells, and a second pump connected to the region for culturing cells. The first pump, the region for culturing cells, and the second pump form a line connected in this order, the first pump and the second pump can move a fluid in a forward direction and a reverse direction, respectively, independently, and pressure when moving the fluid is variable independently.

## Description

### TECHNICAL FIELD

The present invention relates to an apparatus for culturing cells and a method of culturing cells using the same.

### BACKGROUND ART

Improving the functionality of cells used as raw materials for tissues and organs in regenerative medicine and increasing production efficiency has become one of the most important issues in the development of regenerative medicine. Naturally, automated culturing apparatuses used therein are required to reduce the number of human operations and operational errors, thereby achieving cost reduction.

Since it has been reported that cells forming tissues and organs undergo rapid cell proliferation, differentiation, and tissue regeneration/reorganization when cultured under an environment similar to that in a living body, development of an apparatus capable of achieving a culture environment close to a living body is strongly desired, and research in a new field called mechanobiology is becoming active. Users who pay attention to this technology have come to strongly desire the practical application of a function capable of simultaneously applying a plurality of physical and chemical stimuli to an automated culturing apparatus like a living body in order to improve cell functionality and increase production efficiency. However, among apparatuses currently deployed in the market, there is no automated culturing apparatus equipped with a function of simultaneously applying a plurality of physical and chemical stimuli like a living body. Therefore, expensive reagents are relied upon to increase the speed of proliferation and differentiation, and running costs are soaring.

### PRIOR ART DOCUMENTS

### PATENT DOCUMENT

| | |
|---|---|
| PATENT DOCUMENT 1 | JP 5866663 B |
| PATENT DOCUMENT 2 | WO 2017-111148 |

### SUMMARY OF INVENTION

### PROBLEMS TO BE SOLVED BY THE INVENTION

An object of the present invention is to provide an apparatus for culturing cells capable of applying one or a plurality of physical and/or chemical stimuli to cells being cultured, and a method of culturing cells using the same.

### MEANS FOR SOLVING THE PROBLEMS

As a result of intensive studies, the inventors of the present application found that the above object can be achieved by arranging pumps on both sides of a region for culturing cells, respectively, and employing, as these pumps, pumps capable of moving a fluid in a forward direction and a reverse direction independently of each other, and having variable pressures when moving the fluid independently of each other, thereby completing the present invention.

That is, the present invention provides the following.
(1) An apparatus for culturing cells comprising a region for culturing cells, a first pump connected to the region for culturing cells, and a second pump connected to the region for culturing cells, wherein the first pump, the region for culturing cells, and the second pump form a line connected in this order, the first pump and the second pump can move a fluid in a forward direction and a reverse direction, respectively, independently, and pressure when moving the fluid is variable independently.
(2) The apparatus for culturing cells according to (1), further comprising a liquid medium reserve vessel incorporated into the line, wherein a liquid medium contained in the liquid medium reserve vessel is supplied to the region for culturing cells by the first pump and/or second pump.
(3) The apparatus for culturing cells according to (1) or (2), further comprising a cell culture component vessel arranged upstream of the first pump, wherein one or more cell culture components contained in the cell culture component vessel are supplied to the liquid medium contained in the liquid medium reserve vessel by the first pump and/or second pump.
(4) The apparatus for culturing cells according to (3), wherein the one or more cell culture components are supplied in the form of gas to the liquid medium contained in the liquid medium reserve vessel.
(5) The apparatus for culturing cells according to any of (1) to (4), further comprising a cell vessel arranged upstream of the region for culturing cells, wherein cells contained in the cell vessel are supplied to the region for culturing cells by the first pump and/or second pump.
(6) The apparatus for culturing cells according to any of (1) to (5), further comprising a cell recovery column incorporated into the line, wherein cultured cells on the region for culturing cells are peeled off by the first pump and/or second pump, and the peeled off cultured cells are recovered in the cell recovery column.
(7) The apparatus for culturing cells according to (6), further comprising a cell recovery vessel connected to the cell recovery column, wherein cultured cells separated from the cell recovery column are recovered in the cell recovery vessel by the first pump and/or second pump.
(8) The apparatus for culturing cells according to any of (1) to (7), wherein the region for culturing cells is coated with a polymer that makes cells cultured on the region for culturing cells easily peeled off from the region for culturing cells at a low temperature, and cells are cultured on the polymer.
(9) The apparatus for culturing cells according to any of (1) to (8), further comprising an observation camera, a pH meter, a dissolved oxygen meter, a flow meter, and a weight sensor incorporated into the line.
(10) A method of culturing cells comprising culturing cells on the region for culturing cells of the apparatus for culturing cells according to any of (1) to (9).
(11) The method according to (10), wherein the apparatus for culturing cells is the apparatus for culturing cells according to (9), and culturing of cells is performed while measuring a pH and dissolved oxygen of a medium by the pH meter and the dissolved oxygen meter, and appropriately adjusting the pH and dissolved oxygen concentration of the medium.
(12) The method according to (10) or (11), wherein the apparatus for culturing cells is the apparatus for culturing cells according to (9), comprising performing culturing of cells while measuring flow rate of the liquid medium by the flow meter, and performing culturing while appropriately adjusting pressure by the first pump and the second pump to apply pressure, shear stress, or both to the cultured cells.
(13) The method according to any of (10) to (12), wherein the apparatus for culturing cells is the apparatus for culturing cells according to (9), wherein determination of differentiation state, measuring of cell density, prediction of cell proliferation of cells cultured in the region for culturing cells, and measurement of presence/absence and size of bubbles can be performed by the observation camera.
(14) The method according to any of (10) to (13), wherein the apparatus for culturing cells is the apparatus for culturing cells according to (9), wherein the apparatus comprises a function of notifying timing of replacement of consumables by a warning function by measuring weight of the cell culture component vessel by the weight sensor.

### BRIEF DESCRIPTION OF DRAWINGS

FIG. 1 is a schematic view showing a preferred embodiment of the apparatus for culturing cells of the present invention.
FIG. 2 is a diagram schematically showing an example in which AI and IoT are applied to the method of culturing cells of the present invention.

### MODE FOR CARRYING OUT INVENTION

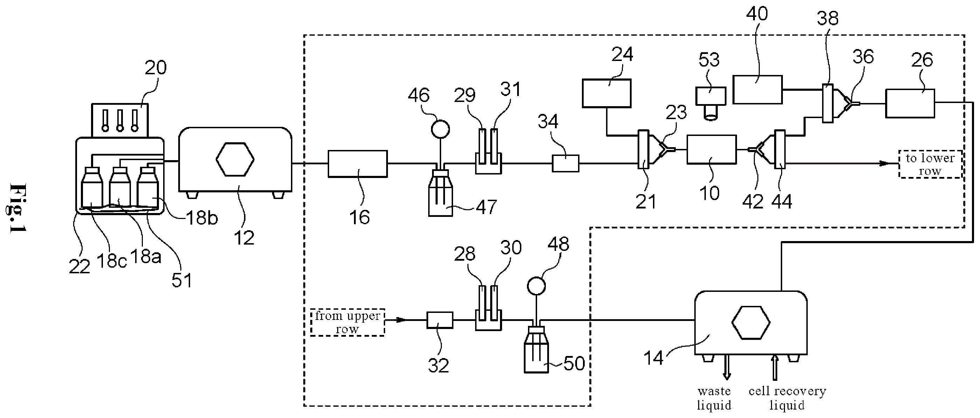

Hereinafter, a preferred embodiment of the present invention will be described with reference to FIG. 1. In the following description, since the flow of the liquid medium flows originating from the liquid medium reserve vessel, in FIG. 1, the side where the first pump 12 is located may be referred to as "upstream", and the side where the second pump 14 is located may be referred to as "downstream".

The apparatus for culturing cells shown in FIG. 1 includes a region for culturing cells 10. A first pump 12 is connected upstream of the region for culturing cells 10. A second pump 14 is connected downstream of the region for culturing cells 10. The first pump 12, the region for culturing cells 10, and the second pump 14 form a line connected in this order. The first pump 12 and the second pump 14 can move a fluid in a forward direction and a reverse direction independently of each other, and pressures when moving the fluid are variable independently of each other.

In the embodiment shown in FIG. 1, in the line, a liquid medium reserve vessel 16 for storing a medium is incorporated between the first pump 12 and the region for culturing cells 10.

The embodiment shown in FIG. 1 further includes cell culture component vessels arranged upstream of the first pump 12. In the illustrated embodiment, three cell culture component vessels 18a, 18b, and 18c are included. The three cell culture component vessels 18a, 18b, and 18c, for example, contain a stimulating drug solution, a buffer solution (PBS), and a liquid medium, respectively. By bubbling gas from a gas mixer 20 into each cell culture component vessel 18a, 18b, 18c, one or a plurality of cell culture components are supplied in the form of gas to the liquid medium contained in the liquid medium reserve vessel 18a, 18b, 18c. It is preferable that the three cell culture component vessels 18a, 18b, and 18c are contained in a refrigerator 22.

Further, in the embodiment shown in FIG. 1, a cell vessel 24 is arranged upstream of the region for culturing cells 10 via a three-way cock 23 and a solenoid valve 21.

Further, in the embodiment shown in FIG. 1, a cell recovery column 26 is further incorporated between the region for culturing cells 10 and the second pump 14 in the line.

Further, in the embodiment shown in FIG. 1, a cell recovery vessel 40 is connected upstream of the cell recovery column 26 via a three-way cock 36 and a solenoid valve 38. The solenoid valve 38 is connected to the region for culturing cells 10 via a three-way cock 42 and a solenoid valve 44 downstream of the region for culturing cells 10.

Further, in the embodiment shown in FIG. 1, two pH meters 28, 29, two dissolved oxygen meters 30, 31, two flow meters 32, 34, and two pressure sensors 46, 48 are incorporated in pressure chambers 47, 50 in the line. The pressure chambers 47, 50 have an effect of suppressing pulsation of a fluid such as a liquid medium sent from the first pump 12 by a damper effect due to gas expansion/compression in the chambers, and by installing two dampers upstream and downstream, it is possible to significantly suppress pulsation compared to installing it at one place.

The region for culturing cells 10 may be a flat surface coated with a solid medium such as agar or agarose gel, but the flat surface is preferably coated with a polymer that makes it easy to make the cells be peeled off from the region for culturing cells at a low temperature. Such a polymer is known, and is described in, for example, Patent Document 2. When the polymer described in Patent Document 2 is coated on a flat surface, the polymer becomes hydrophilic at 20°C or lower and cells are easily peeled off naturally, and it becomes hydrophobic at 25°C or higher so that cells can adhere and proliferate.

Hereinafter, a method of culturing cells using the apparatus for culturing cells of the embodiment shown in FIG. 1 will be described. First, when seeding cells, only the second pump 14 is operated to draw cells from the cell vessel 24 into the region for culturing cells 10. At this time, the solenoid valve 21 is set so that fluid flows from the cell vessel 24 to the region for culturing cells 10.

During cell culture, the first pump 12 and the second pump 14 are operated in a forward direction (flowing fluid from upstream to downstream) to send a medium, a stimulating drug(s), or the like from the cell culture component vessels 18a, 18b, and 18c into the region for culturing cells 10 via the liquid medium reserve vessel 16. At this time, the solenoid valve 21 is set so that fluid flows from the liquid medium reserve vessel 16 to the region for culturing cells 10. A weight sensor 51 is provided below the cell culture component vessels 18a, 18b, and 18c, and it is preferable to have a function of notifying the timing of replacement of consumables by a warning function by measuring the weight. During cell culture, it is preferable to measure the pH and dissolved oxygen of the medium with the pH meters 28, 29 and the dissolved oxygen meters 30, 31, and culture cells while appropriately adjusting the pH and dissolved oxygen concentration of the medium. Further, at this time, it is possible to culture cells while measuring the flow rate of the liquid medium with the flow meters 32, 34, and to culture the cells while applying shear stress to the cultured cells by appropriately adjusting the pressures of the first pump 12 and the second pump 14. Further, it is more preferable that an observation camera 53 is provided above, below, or both of the region for culturing cells to enable determination of differentiation state, cell density, prediction of cell proliferation of the cells being cultured, and to enable measurement of presence/absence and size of bubbles.

When recovering cells, for example, a cold phosphate buffered saline (PBS) stored in the refrigerator 22 is sent to the region for culturing cells 10. At this time, the flow path is switched by the solenoid valves 21 and 44 so as to connect to the cell recovery column 26. As described above, the region for culturing cells 10 is coated with a polymer from which cells are peeled off at 20°C or lower, and the peeled cells are accumulated in the cell recovery column 26. When it is difficult to peel off, the first pump 12 and the second pump 14 are repeatedly operated in the forward direction and the reverse direction to promote peeling of cells by water flow.

The cells accumulated in the cell recovery column 26 are sent to the cell recovery vessel 40 by running a cell recovery liquid in the reverse direction from the second pump 14. At this time, the solenoid valve 38 is set so that fluid flows from the cell recovery column 26 to the cell recovery vessel 40.

According to the above-described apparatus for culturing cells of the present invention, by making the fluid transmission amounts (fluid transmission pressures) of the first pump 12 and the second pump 14 different from each other, it is possible to apply a physical stimulus such as shear stress to cultured cells. Further, by adjusting a desired gas concentration and bubbling into the cell culture component vessels 18b and 18c, one or a plurality of medium components such as gas stimulation and a stimulating drug can be contained in the medium, and a plurality of chemical and/or physical stimuli can be simultaneously applied to cultured cells, making it possible to closer to an environment in a living body. Further, it is possible to automate from cell seeding to cell recovery after culture.

The above-described method of the present invention can be easily optimized by utilizing AI and IoT. More specifically, it is as follows.

### 1. Main functions of AI

(1) Data integration and analysis:
   Integrating data such as pressure, shear stress stimulation, pH, oxygen concentration, cell cycle, cell proliferation, and cell differentiation, and analyzing relationships and correlations. Extracting patterns and trends from an enormous amount of data by utilizing statistical methods and machine learning algorithms. Thereby, the state of the cell culture process can be monitored and accumulated in the cloud.
(2) Pattern prediction and optimization:
   Learning from the accumulated data and constructing a prediction model of the cell culture process. Thereby, the prediction and automatic control for optimizing parameters of pressure, shear stress, drug, gas stimulation, pH, oxygen concentration, cell cycle, cell proliferation, and cell differentiation are possible.
(3) Automatic control and feedback loop:
   Cooperating sensor information and a control system to collect and analyze data in real time, and automatically control cell culture conditions. Adjusting and controlling various stimulation conditions, pH, and oxygen concentration, thereby effectively promoting cell cycle, cell proliferation, and cell differentiation.
(4) Functions of determination of differentiation state, cell density calculation, prediction of cell proliferation of cells, and measurement of presence/absence and size of bubbles by a camera:
   If correlation with pH, gas, and pressure sensors is known, the culture conditions are further optimized using a correlation coefficient as a trigger.

### 2. Main functions of IoT

(1) Real-time monitoring and remote control:
   Monitoring the cell culture process in real time and enabling remote access to data and control commands. Thereby, the cell culture process can be monitored from a remote location, and control and adjustment can be performed as necessary.
(2) Data sharing:
   A user can select so that the optimal parameters accumulated in a cloud server can be shared among users all over the world, groups, and limited stakeholders.
(3) Real-time alert and remote monitoring:
   Monitoring the state of cell culture from a remote location, detecting abnormalities or important events of the cell culture process from various pieces of sensor information, providing an early warning of a problem by notification by an alert in real time, and taking an appropriate measure as necessary.
(4) Replacement/maintenance warning function for consumables (medium and reagents): By adding a replacement and warning function for consumables with a weight sensor for a medium or a reagent, information obtained by IoT is further enriched.

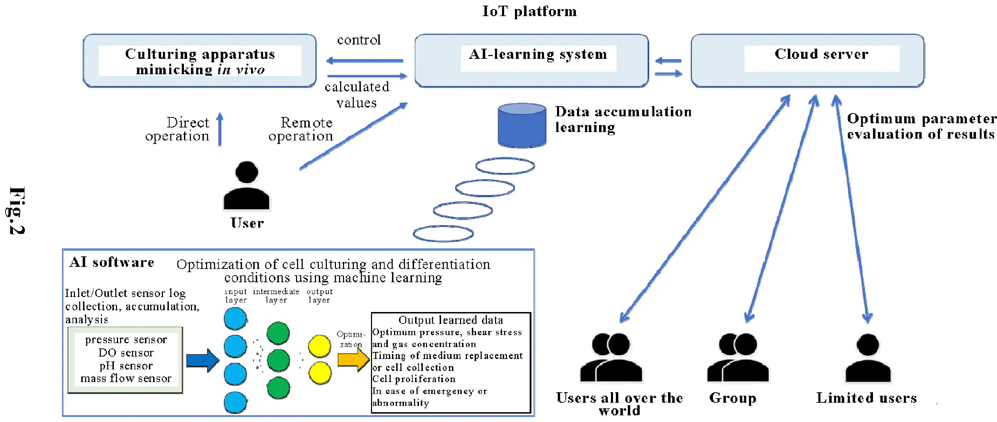

FIG. 2 shows a schematic summary of the utilization methods of the AI and IoT described above.

### REFERENCE SIGNS LIST

10 region for culturing cells
12 first pump
14 second pump
16 liquid medium reserve vessel
18a, 18b, 18c cell culture component vessel
20 gas mixer
21, 38, 44 solenoid valve
22 refrigerator
23, 36, 42 three-way cock
24 cell vessel
26 cell recovery column
28, 29 pH meter
30, 31 dissolved oxygen meter
32, 34 flow meter
46, 48 pressure sensor
47, 50 pressure chamber
51 weight sensor
52 bubble sensor
53 observation camera

## Claims

1. An apparatus for culturing cells comprising a region for culturing cells, a first pump connected to said region for culturing cells, and a second pump connected to said region for culturing cells, wherein said first pump, said region for culturing cells, and said second pump form a line connected in this order, said first pump and said second pump can move a fluid in a forward direction and a reverse direction, respectively, independently, and pressure when moving the fluid is variable independently.

2. The apparatus for culturing cells according to claim 1, further comprising a liquid medium reserve vessel incorporated into said line, wherein a liquid medium contained in said liquid medium reserve vessel is supplied to said region for culturing cells by said first pump and/or second pump.

3. The apparatus for culturing cells according to claim 1 or 2, further comprising a cell culture component vessel arranged upstream of said first pump, wherein one or more cell culture components contained in said cell culture component vessel are supplied to the liquid medium contained in said liquid medium reserve vessel by said first pump and/or second pump.

4. The apparatus for culturing cells according to claim 3, wherein said one or more cell culture components are supplied in the form of gas to the liquid medium contained in said liquid medium reserve vessel.

5. The apparatus for culturing cells according to claim 3, further comprising a cell vessel arranged upstream of said region for culturing cells, wherein cells contained in said cell vessel are supplied to said region for culturing cells by said first pump and/or second pump.

6. The apparatus for culturing cells according to claim 3, further comprising a cell recovery column incorporated into said line, wherein cultured cells on said region for culturing cells are peeled off by said first pump and/or second pump, and the peeled off cultured cells are recovered in said cell recovery column.

7. The apparatus for culturing cells according to claim 6, further comprising a cell recovery vessel connected to said cell recovery column, wherein cultured cells separated from said cell recovery column are recovered in said cell recovery vessel by said first pump and/or second pump.

8. The apparatus for culturing cells according to claim 6, wherein said region for culturing cells is coated with a polymer that makes cells cultured on said region for culturing cells easily peeled off from said region for culturing cells at a low temperature, and cells are cultured on said polymer.

9. The apparatus for culturing cells according to claim 3, further comprising an observation camera, a pH meter, a dissolved oxygen meter, a flow meter, and a weight sensor incorporated into said line.

10. A method of culturing cells comprising culturing cells on said region for culturing cells of the apparatus for culturing cells according to claim 1 or 2.

11. The method according to claim 10, wherein said apparatus for culturing cells is the apparatus for culturing cells according to claim 9, and culturing of cells is performed while measuring a pH and dissolved oxygen of a medium by said pH meter and said dissolved oxygen meter, and appropriately adjusting the pH and dissolved oxygen concentration of the medium.

12. The method according to claim 10, wherein said apparatus for culturing cells is the apparatus for culturing cells according to claim 9, comprising performing culturing of cells while measuring flow rate of said liquid medium by said flow meter, and performing culturing while appropriately adjusting pressure by said first pump and said second pump to apply pressure, shear stress, or both to said cultured cells.

13. The method according to claim 10, wherein said apparatus for culturing cells is the apparatus for culturing cells according to claim 9, wherein determination of differentiation state, measuring of cell density, prediction of cell proliferation of cells cultured in said region for culturing cells, and measurement of presence/absence and size of bubbles can be performed by said observation camera.

14. The method according to claim 10, wherein said apparatus for culturing cells is the apparatus for culturing cells according to claim 9, wherein said apparatus comprises a function of notifying timing of replacement of consumables by a warning function by measuring weight of said cell culture component vessel by said weight sensor.
